# FASCICULE DE BREVET EUROPEEN

(11) **EP 1 056 481 B1**
(45) Date de publication et mention de la délivrance du brevet: **24.10.2001**
(21) Numéro de dépôt: 99903726.0
(22) Date de dépôt: 04.02.1999
(51) Int. Cl.: A61L 2/20

(54) **PROCEDE ET DISPOSITIF POUR LA STERILISATION DES CORPS CREUX**
VERFAHREN UND ANORDNUNG ZUR STERILISATION VON HOHLKÖRPERN
METHOD AND DEVICE FOR STERILISING HOLLOW BODIES

(30) Priorité: 16.02.1998 FR 9801937
(43) Date de publication de la demande: 06.12.2000
(73) Titulaire: SIDEL S.A., 76053 Le Havre Cédex (FR)
(72) Inventeur: FEUILLOLEY, Guy Sidel S.A., 76053 Le Havre Cedex (FR); BERNARD, Véronique Sidel S.A., 76053 Le Havre Cedex (FR)
(74) Mandataire: Siloret, Patrick
(86) Numéro de dépôt international: FR9900253
(87) Numéro de publication internationale: WO9940949

(56) Documents cités:
- DE-A- 4 305 478
- US-A- 3 795 483
- US-A- 4 992 247

## Description

L'invention concerne un procédé pour stériliser des corps creux. Elle s'applique tout particulièrement, quoique non exclusivement, à la stérilisation de récipients entre leur fabrication et leur remplissage, ou bien encore à la stérilisation de préformes ou d'ébauches de récipients lors de processus de fabrication de récipients stériles. Elle concerne encore un dispositif et une installation pour la mise en oeuvre du procédé.

Par stérilisation, au sens de la présente invention, il faut entendre toute procédure tendant à détruire ou réduire des éléments (microbes, germes, levures, etc...) qui, s'ils se développaient, rendraient le contenu du corps creux impropre à l'usage ou à la consommation auquel il est destiné. Il ne faut donc pas nécessairement entendre un degré "zéro" de contamination par un type d'élément ou un autre, mais un degré acceptable en fonction du contenu, de son usage, de sa durée souhaitée de conservation ou d'autres paramètres encore. Ainsi, selon le degré de contamination restant, on parle de corps creux propres ou stériles.

De nombreux procédés ont été envisagés pour l'obtention de corps creux stériles. Mais il ne donnent pas satisfaction pour diverses raisons.

Typiquement, le principe de base est d'injecter un fluide de stérilisation dans le corps creux, voire à l'extérieur si on désire que non seulement l'intérieur mais encore l'extérieur soient stérilisés.

C'est le fluide employé, et le mode opératoire appliqué, qui déterminent le degré de stérilisation atteint.

Des procédés connus consistent à immerger le corps creux, ou à le remplir avec un liquide stérilisant. Ces procédés nécessitent un vidage, puis un séchage des corps creux, d'une part; ils entraînent une forte consommation de fluide ou imposent encore un recyclage d'importants volumes de liquide de stérilisation, d'autre part.

Le vidage, comme le séchage, alors que le corps a été complètement rempli, sont des opérations délicates : il ne faut pas que ces opérations soient la cause d'une nouvelle contamination.

D'autre part, le recyclage du liquide, si on veut réduire la consommation, nécessite une installation complexe.

D'autres procédés consistent en l'emploi de gaz stérilisants. Les installations pour la mise en oeuvre sont complexes et consommatrices. Ainsi, par exemple, on a envisagé de fabriquer des récipients stériles en matière plastique, tels que des bouteilles, en insufflant de l'air stérile ou de l'air additionné d'agents stérilisants. L'obtention d'air stérile est une opération qui nécessite des moyens d'autant plus lourds que les volumes nécessaires au soufflage sont importants. Il faut en effet se rappeler que, pour fabriquer des bouteilles en matière plastique, en employant la technique de l'injection-soufflage, les pressions de soufflage sont de l'ordre de 40 bars, ce qui signifie que la fabrication d'un récipient d'un litre entraîne la consommation de 40 litres d'air. Par ailleurs, après soufflage, il faut dégazer les récipients. Selon la nature de l'agent stérilisant employé, il peut être impossible d'envisager de le relâcher dans l'atmosphère, d'où la nécessité, là encore, d'une installation complexe de recyclage.

On a alors envisagé des procédés qui consistent à atomiser un agent de stérilisation liquide (tel que du péroxyde d'hydrogène, de l'acide péracétique, ou autre) et à l'appliquer sous forme de gouttelettes sur les surfaces à stériliser du matériau d'emballage.

Les procédés de ce genre et les dispositifs correspondants connus jusqu'alors ne sont pas adaptés en ce sens qu'ils ne permettent pas d'assurer une répartition de l'agent de stérilisation sur l'ensemble de la surface interne, encore appelée paroi intérieure, du corps creux lorsque c'est essentiellement cette surface qui doit être stérilisée; par ailleurs, les dispositifs connus ne sont pas "universels" en ce sens qu'ils ne permettent pas le traitement de corps creux ayant des formes distinctes les uns des autres, ou bien encore qu'ils ne sont pas adaptés au traitement de tout type de matière.

Ainsi, par exemple, le dispositif décrit dans le brevet des Etats-Unis d'Amérique n° US-A-4 631 173 ne permet pas d'assurer une répartition homogène de l'agent, car il utilise une surface chauffée sur laquelle l'agent de stérilisation est projeté avec violence. Au contact de cette surface, l'agent se vaporise, d'une part, et est renvoyé vers les surfaces à stériliser, d'autre part. Il est nécessaire d'orienter la surface chauffée pour que l'agent soit dispersé dans le corps creux.

Il faut donc adapter la configuration et l'orientation de la surface chauffée au volume et à la forme du corps creux. Si le corps creux est trop profond, il n'est pas certain que l'agent de stérilisation sera renvoyé vers les surfaces qui en sont éloignées.

Ce dispositif n'est pas adapté à la stérilisation de bouteilles ou de préformes (ou ébauches) de bouteilles, car leur ouverture n'est pas assez grande pour que le produit dispersé puisse être dirigé à l'intérieur de ce type de corps creux, sauf à avoir une surface chauffante de très faibles dimensions pour qu'elle puisse être introduite dans l'ouverture du récipient. Mais dans ce cas, il serait difficile d'amener l'agent stérilisant au contact même de cette surface. Par ailleurs, l'énergie calorifique nécessaire à la vaporisation serait susceptible de détériorer le corps creux (bouteille ou préforme) lorsqu'il est réalisé en une matière thermosensible.

Le document DE-A-4 305 478 décrit un procédé de stérilisation d'un corps creux, comprenant l'injection d'un agent de stérilisation vaporisé avec des moyens d'injection débouchant dans le corps creux.

Le procédé décrit dans la demande de brevet allemand DE-A-43 05 478 ne permet pas d'assurer une répartition homogène de l'agent de stérilisation, et n'est pas adapté à tout type de matériau constitutif du corps creux.

Il ne permet pas d'assurer une répartition homogène de l'agent de stérilisation, car ce dernier est injecté par l'intermédiaire d'une buse qui débouche en regard du fond du corps creux. L'agent est introduit dans la buse en étant mélangé avec de la vapeur d'eau afin de ressortir, en théorie, en phase vapeur dans le corps creux. Mais, si le tube se refroidit, le mélange vapeur d'eau - agent de stérilisation condense, et ce sont alors des gouttes, qui tombent au fond du corps, au lieu d'un nuage de vapeur. D'où un risque d'accumulation de gouttes sur le fond et non pas une remontée d'agent sur les parois.

Pour réduire les risques d'accumulation et/ou éviter une condensation de l'agent au fond du récipient, il est prévu que les corps creux soient réchauffés, ce qui permet de réactiver la vaporisation de l'agent.

Ceci n'assure pas pour autant que l'agent vaporisé va correctement se répartir sur l'ensemble des parois du corps creux.

Par ailleurs, cette méthode de réchauffage rend la mise en oeuvre impossible avec certains types de matériaux, notamment les matériaux thermosensibles (plastiques) ou les matériaux isolants. En effet, un corps creux en matière thermosensible risque d'être détérioré par le procédé (risque de déformation par rétraction de la matière notamment); un matériau isolant augmentera considérablement le temps de traitement, puisque l'apport de chaleur pour réchauffer le corps creux s'effectue de l'extérieur; à la limite, il rendra impossible la mise en oeuvre du procédé tant le temps nécessaire au réchauffage sera important.

Cette méthode nécessite encore de modifier notablement l'installation (changement de l'enceinte de réchauffage) en cas de changement de formes ou de dimensions du corps creux à stériliser.

Un autre inconvénient de ce procédé est qu'un manque ou une absence d'agent de stérilisation, en cas de défaillance d'un système de contrôle, ne sera pas détecté, puisque l'agent est normalement mélangé à de la vapeur d'eau. En cas d'injection de vapeur d'eau seulement, l'absence d'agent stérilisant ne pourra pas être aisément détectée par un observateur extérieur.

D'autres approches ont été faites, qui consistent notamment à propulser l'agent stérilisant dans le corps creux à l'aide d'un gaz comprimé.

Cette solution ne permet pas d'assurer une répartition correcte de l'agent, puisque, lors de son introduction sous pression, il se crée des noeuds et des ventres de compression, des phénomènes turbulents, ou autres obstacles à ladite répartition.

L'invention a donc pour objet un procédé et un dispositif qui ne présentent pas l'un ou l'autre les inconvénients précités, à savoir qui permettent : une répartition sur l'ensemble de la surface de l'agent de stérilisation; une possibilité d'application, sans modification notable du dispositif, en cas de changement de corps creux à stériliser; une application à tout type de corps creux (bouteilles, pots, flacons, vasques, tubes...); une application à tout type de matériau; une détection aisée d'une absence d'agent stérilisant.

Selon l'invention, un procédé pour stériliser des corps creux, notamment des récipients ou des préformes de récipients, en déposant un agent stérilisant préalablement vaporisé sur les surfaces à stériliser, est caractérisé en ce qu'il consiste à provoquer un courant gazeux d'entraînement de l'agent vaporisé vers l'ensemble des surfaces à stériliser afin de le répartir sur la totalité desdites surfaces, selon les revendications 1 et 2.

Ainsi, en entraînant, c'est-à-dire en guidant l'agent sur les surfaces, on en obtient une répartition correcte contrairement aux dispositifs où on projette ou insuffle l'agent à l'intérieur. Un simple entraînement de l'agent, après sa vaporisation, rend le procédé utilisable avec tout type de matériau puisque, notamment, il n'y a plus besoin de chauffer les corps creux; comme il sera expliqué plus loin, le procédé peut être mis en oeuvre avec un dispositif relativement simple utilisable pour tout type de corps creux quelles que soient leurs formes ou dimensions, et sans qu'il y ait besoin de le modifier notablement lorsque la forme et/ou les dimensions du corps sont changées; enfin, l'absence d'agent est aisément détectable: il suffit de constater l'arrivée ou non de l'agent au moment de la vaporisation.

Un autre avantage est que l'entraînement de l'agent par un courant d'entraînement gazeux permet de réaliser la stérilisation quelle que soit la disposition du corps creux, c'est-à-dire quelle que soit la position de son ouverture (haut, bas ou autre).

La vaporisation de l'agent est provoquée à l'extérieur du corps creux, à proximité de son ouverture, et le courant d'entraînement est réalisé en provoquant une aspiration de l'agent stérilisant vaporisé à l'aide de moyens d'aspiration débouchant à l'opposé de l'ouverture dans le corps creux, selon la revendication 1.

Alternativement dans un procédé destiné à la stérilisation de tubes ouverts à leurs deux extrémités, l'aspiration est provoquée à l'aide d'un dispositif agissant du côté de l'extrémité du tube opposée à celle à proximité de laquelle est réalisée la vaporisation, selon la revendication 2.

Selon une autre caractéristique du procédé de l'invention, les phases de vaporisation et d'introduction de l'agent dans le corps creux sont précédées d'une phase de retrait des particules ou éléments non adhérents présents dans le corps creux, telles les poussières introduites lors du stockage.

Selon une autre caractéristique, la phase de vaporisation est suivie, après un temps de contact, d'une phase de retrait (séchage) de l'agent stérilisant restant.

D'autres caractéristiques et avantages de l'invention apparaîtront à la lecture de la description qui suit, faite en regard des figures annexées, sur lesquelles :
- la figure 1 est un schéma de principe d'un dispositif pour stériliser des corps creux conformément à l'invention;
- la figure 2 est un schéma de principe d'un dispositif de retrait de l'agent stérilisant après un temps de contact déterminé;
- la figure 3 est une vue schématique, de dessus, d'une variante d'un dispositif conforme à l'invention;
- la figure 4 est une vue schématique d'un dispositif utilisé pour le retrait de l'agent stérilisant;
- la figure 5 est un schéma bloc d'une installation utilisant l'invention;
- la figure 6 est un schéma d'une variante des dispositifs des figures 1 à 4.

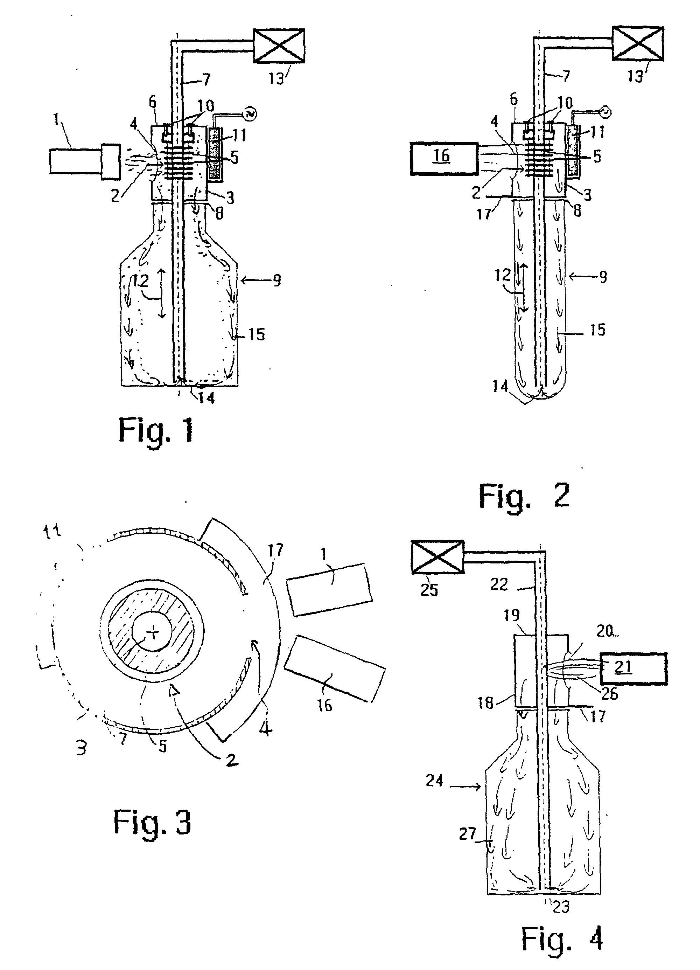

Le dispositif représenté à la figure 1 comporte un injecteur ou pulvérisateur 1 d'agent stérilisant vaporisable, tel que du péroxyde d'hydrogène, de l'acide péracétique, ou tout autre agent adapté. L'injecteur est relié d'une part à une réserve d'agent, non représentée, et à des moyens de commande pour son ouverture et sa fermeture, d'autre part.

L'injecteur peut être mécanique, électromécanique, pneumatique ou de tout autre type approprié.

La sortie du liquide stérilisant de l'injecteur est dirigée vers un évaporateur 2 enfermé dans un carter 3, cylindrique dans l'exemple illustré. Une ouverture 4 est ménagée dans la paroi cylindrique du carter en regard de la sortie de l'injecteur 1 pour permettre la pulvérisation du liquide sur l'évaporateur 2.

Dans le mode de réalisation illustré, l'évaporateur 2 est constitué par un cylindre en un matériau bon conducteur thermique, et pourvu, sur sa périphérie, d'ailettes 5 en forme d'excroissances annulaires. De préférence, les ailettes sont régulièrement réparties sur la hauteur du cylindre formant le corps de l'évaporateur.

Le diamètre extérieur de l'évaporateur est inférieur au diamètre intérieur du carter 3 pour qu'après évaporation, l'agent de stérilisation puisse circuler comme il sera expliqué plus loin.

Le carter 3 est fermé, à son extrémité supérieure sur la figure, par un couvercle 6 percé en son centre d'une ouverture pour le passage d'un tube 7 creux coulissant.

De préférence, comme -illustré sur la figure, le tube 7 passe au travers d'un orifice axial, non référencé, de l'évaporateur 2.

L'extrémité inférieure du carter est destinée à se trouver en regard de l'ouverture 8 du corps creux 9 (ici un récipient tel qu'une bouteille) à stériliser. Elle est ouverte et, de préférence, la forme et les dimensions intérieures de l'ouverture de l'extrémité correspondent respectivement à celles de l'ouverture intérieure du corps creux.

L'évaporateur 2 est maintenu en place dans le carter 3 à l'aide de moyens 10 de fixation tels que des vis ou des entretoises disposées à proximité de l'extrémité de l'évaporateur la plus éloignée du corps creux 9, afin de ne pas interférer avec le trajet de l'agent évaporé entre l'évaporateur 2 et le corps creux 9.

Des moyens de réchauffage 11 de l'évaporateur 2 sont prévus pour le porter à une température permettant d'évaporer quasi instantanément l'agent stérilisant lorsqu'il arrive à son contact. Dans l'exemple illustré, ces moyens 11 sont constitués par une résistance chauffante disposée dans l'épaisseur de la paroi du carter 3, de façon à réchauffer ce dernier et à réchauffer l'évaporateur 2 par conduction thermique au travers du matériau constitutif du carter 3 et de l'évaporateur 2 et/ou par convection dans l'espace libre entre la paroi intérieure du carter 3 et l'évaporateur 2.

De préférence, au moins le tube 7 est coulissant (double flèche 12) pour faciliter la mise en place du corps creux 9 en regard du dispositif. Ainsi, le corps creux 9 peut être amené en regard du dispositif par simple translation latérale en effaçant le tube du trajet d'amenée du corps creux. Il est ainsi possible d'utiliser, pour assurer la mise en place du corps creux, des systèmes de transfert connus en soi, tels que des mécanismes à pinces de transfert, montés sur des bras articulés, ou encore des mécanismes à roues ou plateaux de transfert comportant des encoches ou alvéoles de guidage des corps creux.

Alternativement, il pourrait être envisagé que la mise en place du corps creux s'effectue en lui faisant subir une translation axiale, le tube 7 restant fixe.

Dans une mise en oeuvre, seul le tube 7 est coulissant. Il coulisse donc relativement au corps creux, et à l'ensemble constitué par l'évaporateur 2 et le carter 3 lors de la mise en place ou du retrait du corps creux.

Dans une variante, c'est le tube 7 et l'ensemble évaporateur 2, carter 3 qui sont translatés axialement lors de la mise en place ou du retrait du corps creux 9.

L'extrémité du tube 7 située du côté du couvercle 6 du carter 3 est reliée à une source d'aspiration 13. Lorsque le corps creux 9 est en place, comme illustré sur la figure 1, l'extrémité du tube débouchant dans le corps creux 9 est à proximité du fond 14 de ce dernier.

Le fonctionnement du dispositif est le suivant.

L'évaporateur 2 est chauffé par l'intermédiaire des moyens de chauffage 11, de façon que sa température soit suffisante pour provoquer une évaporation immédiate de l'agent stérilisant lorsque ce dernier, projeté par l'injecteur 1, arrive à son contact.

A titre d'exemple, lorsque l'agent est du péroxyde d'hydrogène, l'évaporateur 2 est porté à une température comprise entre 100°C et 400°C, de préférence de l'ordre de 150 à 200°C.

L'ouverture 8 du corps creux 9 est placée en regard de l'ouverture du carter, sans toutefois venir à son contact, pour qu'un courant d'air puisse se créer entre l'orifice 4 du carter 3 et l'interstice subsistant entre le récipient et le carter.

Par ailleurs, le fait de maintenir un écartement entre le carter et le corps creux évite la détérioration de ce dernier lorsqu'il est réalisé en matériau thermosensible.

Des tests ont mis en évidence qu'un écartement compris entre 0,1 mm et 5 mm procurait de bons résultats, et que les meilleurs résultats étaient obtenus en laissant subsister un écartement compris entre 0,5 et 3 mm.

De préférence, le dispositif est maintenu dans une ambiance d'air stérile en surpression pour éviter que des particules ou poussières supplémentaires ne pénètrent dans les corps creux à stériliser. Pour cela, des moyens connus sont utilisés, tels qu'une chambre d'isolation sous flux laminaire d'air stérile dans laquelle est installé le dispositif.

Après que le dispositif et le corps creux 9 ont été mis en position relative correcte l'un par rapport à l'autre, de l'agent stérilisant est pulvérisé ou injecté par l'injecteur ou pulvérisateur 1 sur l'évaporateur 2 au travers de l'orifice 4 ménagé dans le carter 3. La haute température de l'évaporateur 2 provoque une vaporisation immédiate de l'agent stérilisant, et l'aspiration engendrée par les moyens 13 au travers du tube 7 débouchant à proximité du fond 14 du corps creux 9 crée un courant d'entraînement de l'air contenu dans le corps creux, suivi d'un entraînement des vapeurs de l'agent stérilisant qui sont alors dirigées à l'intérieur du corps creux 9 et viennent se déposer sur la surface interne du corps creux. Ce courant est schématisé par les flèches 15 sur la figure.

De plus, étant donné que le corps creux est à température ambiante, l'agent stérilisant se condense en contactant la surface interne du corps creux.

Après un temps de contact déterminé, l'agent stérilisant contenu dans le corps creux est retiré.

Des essais, menés sur des préformes de récipients en matière plastique (PET) ou sur les récipients mêmes montrent qu'un temps de contact compris entre 2 et 6 secondes permet d'obtenir un degré d'aseptie compatible avec les normes alimentaires en vigueur en utilisant comme agent de stérilisation du péroxyde d'hydrogène concentré à 35 %.

L'aspiration au travers du tube 7 peut être permanente ou séquentielle. Si elle est séquentielle, il est par contre important qu'elle commence au plus tard au moment de l'injection afin que tout l'agent stérilisant injecté et vaporisé soit aspiré vers le corps creux. Une conséquence d'une aspiration commençant avec un léger retard sur l'injection serait le risque de perte d'une certaine quantité d'agent, par évaporation vers l'extérieur, notamment par l'orifice 4. Ce risque de perte n'existe plus si l'aspiration commence au moment de l'injection.

En outre, toujours si elle est séquentielle, elle doit se prolonger au moins jusqu'à ce que la totalité de la surface à traiter soit revêtue d'agent stérilisant.

A l'issue du temps de contact susmentionné, le corps creux est stérile. Il convient cependant de retirer l'agent stérilisant, par exemple en asséchant le corps creux lorsque l'agent est liquide.

Pour ce faire, il est possible soit de souffler un gaz stérile tel de l'air stérile sec ou chaud dans le corps creux soit, de préférence, d'entraîner de l'air chaud à l'intérieur du corps creux par aspiration. En effet, l'insufflation d'air chaud est difficile dans le cas de corps creux ayant une géométrie particulière, par exemple dans le cas des bouteilles où le flux d'air se dirige préférentiellement vers le fond, mais ne vient pas au contact de l'ensemble des parois.

Par ailleurs, un avantage de la solution consistant à entraîner l'air chaud est qu'il est possible d'utiliser le même dispositif que celui employé pour la vaporisation et le dépôt d'agent stérilisant, en substituant à l'injecteur 1 un générateur de chaleur 16 tel que représenté sur la figure 2 (non conforme à la revendication 14).

Dans le cas d'emploi d'air chaud, le générateur de chaleur 16 peut être une buse d'air chaud à haute température ou un brûleur. La chaleur ou la flamme est dirigée vers l'orifice 4 et simultanément une aspiration est créée dans le corps creux (ici une préforme de récipient) pour canaliser la flamme ou la chaleur vers les parois.

L'emploi d'une flamme ou d'un générateur d'air chaud n'est pas contre-indiqué pour le séchage de corps creux en matière thermosensible, tels que des récipients ou des préformes de récipients en matière thermoplastique.

En effet, le temps d'exposition à la chaleur est très faible. Compte tenu de l'inertie thermique du matériau, l'air chaud dirigé a uniquement une fonction de balayage ou de séchage du corps creux.

Toutefois, lorsque le corps creux à stériliser est en matière thermoplastique, il est préférable de prévoir des moyens pour empêcher un transfert direct entre la sortie du générateur 16 de chaleur et l'extérieur du corps creux 9.

Ces moyens peuvent être constitués par une plaque 17 de protection de l'ouverture du corps creux entourant au moins partiellement le carter 3 et disposée entre l'orifice 4 de passage de l'air chaud de la flamme et l'ouverture du carter 3 côté corps creux 9, comme visible sur la figure 2.

Alternativement, il pourrait être prévu une coiffe en forme de cloçhe recouvrant plus largement l'ouverture du corps creux.

Sur la figure 3, on a représenté schématiquement, en vue en coupe de dessus au niveau de l'orifice 4, un arrangement possible d'un dispositif unique servant à la fois au dépôt d'agent stérilisant et au séchage.

A la fois la sortie d'un injecteur 1 et celle d'un .générateur d'air chaud 16 sont dirigées vers l'orifice 4 du carter 3. Dans ce cas, il faut que le générateur 16 d'air chaud soit du type séquentiel pour que la chaleur (flamme ou air chaud) ne soit pas appliquée pendant les phases d'injection / vaporisation / contact de l'agent de stérilisation.

De préférence, la phase de séchage du corps creux s'effectue en deux étapes : une première où la chaleur (flamme ou air chaud) est appliquée dans le carter, l'aspiration étant active; et une seconde ou l'aspiration continue alors que la chaleur n'est plus appliquée, de façon à ce que tous les résidus de vapeur soient évacués.

A titre indicatif, on parvient à assécher des préformes de récipients en matière thermoplastique en appliquant une flamme pendant 1 à 3 secondes à l'orifice 4 et en continuant l'aspiration entre 2 et 6 secondes.

Sur la figure 4, on a représenté en coupe schématique un dispositif prévu spécifiquement pour l'assèchement des corps creux (non conforme à la revendication 14).

La différence principale avec le dispositif des figures 1 à 3 est qu'il ne comporte plus d'évaporateur.

Il comporte un carter 18 en forme de tube cylindrique fermé 19 à l'une de extrémités et ouvert à l'autre. Le carter est percé d'un orifice 20 en regard duquel se trouve un générateur 21 de chaleur, un brûleur dans l'exemple.

Une tige creuse 22 traverse le carter de façon que l'une 23 de ses extrémités se trouve à proximité du fond du corps creux 24. L'autre extrémité de la tige est reliée à un dispositif 25 d'aspiration.

Lorsque la chaleur, ici une flamme 26, est appliquée en même temps que l'aspiration, il se crée un flux (flèches 27) aspirant la flamme et les vapeurs d'agent stérilisant et assèchant la surface intérieure du corps creux.

De préférence, la phase d'injection/vaporisation d'agent est précédée d'une phase de retrait du corps creux des particules telles que poussières ou autres éléments non adhérents.

Cete phase peut être effectuée par aspiration et/ou soufflage ou toute autre méthode appropriée.

Dans une mise en oeuvre, on utilise un dispositif semblable à celui utilisé pour le retrait des traces d'agent stérilisant et présenté en figure 4 à cette différence près que le dispositif ne comporte pas de générateur d'air chaud. Le dispositif comporte donc des moyens d'aspiration 22,23,25 et un carter 18.

Dans une variante, au lieu d'aspirer par la tige 22, la phase de retrait préalable à l'injection d'agent s'effectue non plus en aspirant par la tige 22, mais en aspirant par le carter 18, via par exemple l'orifice 20. Dans ce cas, la tige 22 permet d'amener l'air compensant la dépression créée lors de l'aspiration.

Dans une mise en oeuvre, l'ensemble des phases évoquées ci-avant est réalisé sur un seul et même dispositif, tel celui présenté sur la figure 3, et comportant donc un évaporateur, des moyens d'aspiration, des moyens d'injection d'agent et un générateur d'air chaud, c'est-à-dire l'ensemble des moyens présentés plus en détail sur les figures 1,2 et 4.

Cependant, pour des raisons d'efficacité des divers traitements à réaliser, il est souhaitable de réaliser au moins la phase préalable de dépoussiérage dans un dispositif distinct de celui ou de ceux utilisé(s) pour les phases ultérieures de stérilisation c'est-à-dire injection / vaporisation / contact et de séchage.

En effet, la première phase consiste à récupérer des poussières ou autres particules, alors que, lors des suivantes, ce sont des gaz ou vapeurs qui sont aspirés. Les poussières ou autres particules doivent être récupérées et mises au rebut alors que les gaz ou vapeurs d'agent stérilisant peuvent être recyclés. Donc, si on veut organiser un recyclage des vapeurs ou gaz, il est nécessaire d'avoir des moyens d'aspiration distincts, et il est souhaitable de différencier les dispositifs respectifs.

En outre, l'utilisation d'un dispositif unique n'est pas adaptée lorsque le dispositif doit avoir des cadences élevées, par exemple lorsque les corps creux à stériliser sont des préformes de récipients (bouteilles ou autres) en matière plastique, amenées au défilé à l'entrée d'une machine de soufflage de récipients, ou des récipients en sortie de fabrication.

Dans un tel cas, il est souhaitable et préférable de séparer l'ensemble des phases et de les réaliser en faisant défiler les corps creux dans des étages distincts permettant un traitement au défilé.

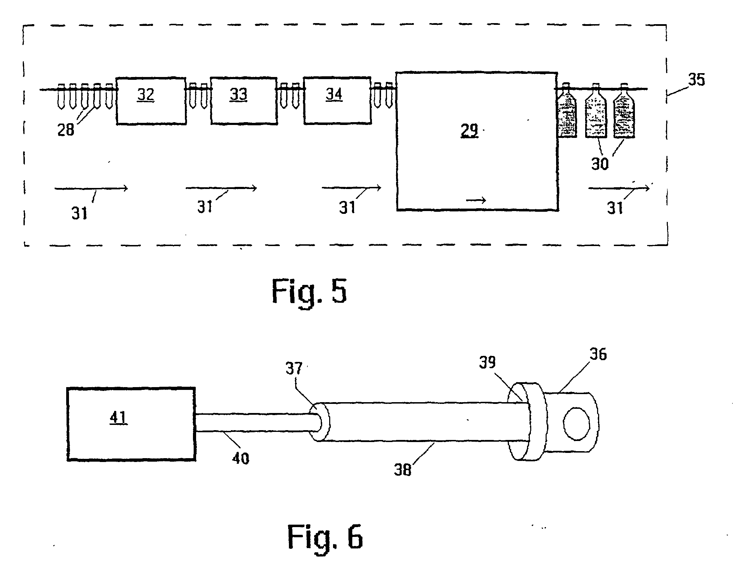

La figure 5 illustre le schéma de principe d'une installation permettant de réaliser l'ensemble des phases sus-mentionnées lors du traitement de préformes 28 en vue de leur stérilisation en amont d'une machine 29 de soufflage/remplissage de récipients 30.

Les préformes 28, défilant en continu dans le sens des flèches 31 sont successivement amenées dans un premier étage 32 de retrait (dépoussiérage ou autre). Cet étage comporte des moyens, non représentés, pour traiter au défilé plusieurs préformes.

Puis, les préformes sont alors transférées dans un étage 33 agencé pour l'injection, la vaporisation et le contact d'un agent stérilisant. Cet étage comporte un ou plusieurs dispositif(s) conformes à celui de la figure 1.

Ensuite, les préformes sont transférées vers un étage 34 de séchage. Il est à noter que le temps de transfert entre l'état précédent et ce dernier est mis à profit pour prolonger le temps de contact nécessaire à la stérilisation.

Puis, les préformes sont transférées vers la machine 29 de fabrication/remplissage des récipients.

De préférence, la machine 29 et les divers étages de traitement 32,33,34 sont enfermés dans une enceinte stérile sous flux laminaire.

Cette enceinte est schématisée par le trait interrompu 35 sur la figure 5.

Chacun des étages de traitement, respectivement 32,33,34 peut comprendre au moins un dispositif fixe approprié devant lequel les corps creux sont amenés en pas à pas en vue de leur traitement.

Cependant, cette structure n'est pas bien adaptée aux cadences élevées de traitement requises dans nombre d'installations; c'est pourquoi, de préférence, chaque étage est agencé pour permettre un traitement au défilé des corps creux.

Dans une mise en oeuvre, chaque étage comporte des moyens de saisie ou de support des corps creux, associés à des moyens de transport en continu. Il est ainsi possible de traiter les corps creux au fur et à mesure de leur arrivée dans l'installation.

La cadence de traitement est fonction du dimensionnement de chacun des étages et donc du nombre de dispositifs que chacun incorpore.

Dans une variante non représentée, les phases de stérilisation s'effectuent après la fabrication des récipients, par exemple avant leur remplissage. Les divers étages, notamment celui 33 pour la stérilisation proprement dite, sont alors en aval de la sortie de la machine 29. Dans ce cas, la machine 29 peut ne pas être sous flux luminaire d'air stérile; mais il est important qu'au moins les étages 33,34 de stérilisation et de séchage soient, quant à eux, sous flux luminaire.

Sur la figure 6, on a illustré un schéma de principe de "l'invention, lorsqu'elle est appliquée à la stérilisation de corps creux ouverts à deux de leurs extrémités. C'est par exemple le cas de tubes ou de conduits divers.

Dans ce cas, l'aspiration est réalisée non pas à l'aide d'un tube traversant le carter 36, et débouchant à l'extrémité 37 du corps creux 38 opposée à celle 39 près de laquelle se trouve le carter 36 avec les moyens d'injection (non représentés) et/ou les moyens de génération d'air chaud (non représentés), mais elle est réalisée à l'aide d'un tube 40 situé à l'extrémité 37 ouverte du corps creux opposée à celle 39 près de laquelle se trouve le carter 36. Le tube 40 est connecté à des moyens d'aspiration 41.

## Revendications

1. Procédé pour stériliser des corps creux (9) possédant une ouverture (8), du genre consistant à déposer sur les surfaces à stériliser un agent stérilisant préalablement vaporisé, **caractérisé en ce qu'**il consiste à réaliser la vaporisation de l'agent à l'extérieur du corps creux, à proximité de son ouverture, et à provoquer, à l'aide de moyens d'aspiration débouchant dans le corps creux à l'opposé de ladite ouverture, un courant gazeux d'entraînement de l'agent vaporisé vers l'ensemble des surfaces à stériliser, afin de répartir l'agent sur la totalité desdites surfaces.

2. Procédé pour stériliser des corps creux (38) possédant deux ouvertures, du genre consistant à déposer sur les surfaces à stériliser un agent stérilisant préalablement vaporisé, **caractérisé en ce qu'**il consiste à réaliser la vaporisation de l'agent à l'extérieur du corps creux, à proximité de l'une de ses ouvertures, et à provoquer, à l'aide de moyens d'aspiration débouchant dans le corps creux à l'opposé de ladite ouverture, un courant gazeux d'entraînement de l'agent vaporisé vers l'ensemble des surfaces à stériliser, afin de répartir l'agent sur 1a totalité desdites surfaces,

3. Procédé selon l'une des revendications précédentes, **caractérisé en ce que** l'agent stérilisant est choisi parmi les agents disponibles en phase liquide, tels que le péroxyde d'hydrogène, l'acide péracétique ou autre.

4. Procédé selon l'une des revendications précédentes, **caractérisé en ce que** les phases de vaporisation et d'introduction d'agent dans le corps creux sont précédées d'une phase de suppression des particules ou autres éléments non adhérents présents dans le corps creux,

5. Procédé selon la revendication 4, **caractérisé en ce que** la phase de suppression est réalisée par soufflage.

6. Procédé selon la revendication 4, **caractérisé en ce que** la phase de suppression est réalisée par aspiration.

7. Procédé selon l'une des revendications précédentes, **caractérisé en ce que** la phase de vaporisation est suivie, après un temps de contact de l'agent avec les surfaces à stériliser, d'une phase de retrait de l'agent stérilisant restant.

8. Procédé selon la revendication 7, **caractérisé en ce que** la phase de retrait est réalisée par injection d'un agent de retrait à l'intérieur du corps creux.

9. Procédé selon la revendication 8, **caractérisé en ce que** l'agent de retrait est un gaz stérile sec ou chaud.

10. Procédé selon la revendication 9, **caractérisé en ce que** l'agent de retrait est entraîné, par aspiration à l'intérieur du corps creux, à l'aide de moyens d'aspiration agissant dans le corps creux à l'opposé de son ouverture.

11. Procédé selon l'une des revendications 8 ou 9, **caractérisé en ce que** le gaz stérile est de l'air chaud injecté à l'aide d'une buse d'air chaud.

12. Procédé selon la revendication 8, **caractérisé en ce que** la phase de retrait est réalisée à l'aide d'une flamme injectée par un brûleur et entraînée par aspiration à l'intérieur du corps creux, à l'aide de moyens d'aspiration agissant dans ce dernier, à l'opposé de son ouverture.

13. Procédé selon l'une des revendications 8 à 12, **caractérisé en ce que** l'agent de retrait, gaz sec ou chaud ou flamme respectivement, est injecté à l'extérieur du corps creux, à proximité de son ouverture.

14. Dispositif pour la mise en oeuvre du procédé selon l'une des revendications 1 à 13, **caractérisé en ce qu'**il comporte des moyens (1) d'injection d'un agent stérilisant vaporisable, un évaporateur (2) en regard de la sortie des moyens d'injection, des moyens pour provoquer un mouvement relatif des moyens d'aspiration (7,13;40,41) et du corps creux de manière à ce que les moyens d'aspiration débouchent dans le corps creux, de façon à agir dans celui-ci, pour provoquer un courant gazeux d'entraînement de l'agent vaporisé vers les surfaces intérieures d'un corps creux (9;38) lorsque ce dernier est en place relativement au dispositif.

15. Dispositif selon 1a revendication 14, **caractérisé en ce que** l'évaporateur est enfermé dans un carter (3;36) disposé à l'extérieur du corps creux (9; 38) à proximité de l'ouverture de ce dernier, et pourvu : d'une ouverture (4) en regard de la sortie des moyens (1) d'injection; d'une extrémité ouverte ayant une forme et des dimensions intérieures correspondant sensiblement à celles de l'ouverture intérieure du corps creux (9;38).

16. Dispositif selon l'une des revendications 14 ou 15 **caractérisé en ce que** les moyens (7,13;40,41) pour provoquer le courant gazeux sont constitués par un tube (7;40) relié à une source d'aspiration (13;41).

17. Dispositif selon la revendication 16, **caractérisé en ce que** le corps creux étant en forme de récipient, c'est-à-dire ouvert à l'une de ses extrémités et fermé à l'autre, le tube (7) est introduit par l'ouverture du corps creux; le tube possède une extrémité débouchant à proximité du fond du corps creux, et sa seconde extrémité, située du côté du carter (3) est reliée à la source d'aspiration (13).

18. Dispositif selon la revendication 17, **caractérisé en ce que** le tube (7) traverse l'évaporateur (2) et le carter (3).

19. Dispositif selon la revendication 16, **caractérisé en ce que** le corps creux (38) étant un tube ou un conduit ouvert à deux extrémités opposées, l'aspiration est effectuée en disposant le tube (40) d'aspiration à l'extrémité du corps creux opposée à celle où s'effectue l'injection.

20. Dispositif selon l'une des revendications 14 à 19, **caractérisé en ce qu'**il comporte des moyens pour retirer l'agent stérilisant après un temps de contact dans le corps creux.

21. Dispositif selon la revendication 20, **caractérisé en ce que** les moyens comportent un générateur (16;21) de gaz stérile sec ou chaud.

22. Dispositif selon la revendication 21, **caractérisé en ce que** le générateur est un brûleur (21).

23. Dispositif selon l'une des revendications 21 ou 22, **caractérisé en ce que** le générateur (15;21) est disposé à l'extérieur du corps creux, à proximité de son ouverture, et il comporte des moyens pour diriger la chaleur à l'intérieur du corps creux.

24. Dispositif selon la revendication 23, **caractérisé en ce que** les moyens pour diriger la chaleur sont constitués par le tube (7;22;40) d'aspiration dont l'extrémité d'aspiration débouche dans le corps creux dans une zone éloignée de son ouverture, afin d'entraîner la chaleur dans l'ensemble du corps creux.

25. Dispositif selon la revendication 24, **caractérisé en ce que** le corps creux étant un récipient, le tube (7;22) pénètre par l'ouverture de ce dernier et débouche à proximité de son fond.

26. Dispositif selon la revendication 24, **caractérisé en ce que** le corps creux étant un tube ou un conduit, le tube (40) est disposé à l'extrémité du corps creux opposée à celle prés de laquelle se trouve le générateur (16;21) de chaleur.

27. Dispositif selon l'une des revendications 14 à 26, **caractérisé en ce qu'**il comporte des moyens pour effectuer une suppression des poussières ou d'autres particules préalablement à la phase de vaporisation et de mise en place de l'agent stérilisant.

28. Dispositif selon la revendication 27, **caractérisé en ce que** la suppression s'effectue par aspiration et/ou soufflage.

29. Dispositif selon l'une des revendications 14 à 28, **caractérisé en ce qu'**il est parcourable par un flux laminaire (35) de gaz stérile, en surpression, tel de l'air.

30. Installation de fabrication et/ou de remplissage de récipients, **caractérisée en ce qu'**elle comporte un dispositif selon l'une des revendications 14 à 29.

## Patentansprüche

1. Verfahren zur Sterilisation von Hohlkörpern (9) mit einer Öffnung (8) jener Art, bei der man auf die zu sterilisierenden Flächen ein zuvor verdampftes Sterilisationsmittel aufbringt, **dadurch gekennzeichnet, daß** man die Verdampfung des Mittels außerhalb des Hohlkörpers in der Nähe seiner Öffnung durchführt und mit Hilfe von im Hohlkörper gegenüber der Öffnung mündenden Saugvorrichtungen einen Gasstrom zur Mitführung des verdampften Mittels zu allen zu sterilisierenden Flächen erzeugt, um das Mittel auf die gesamten Flächen zu verteilen.

2. Verfahren zur Sterilisation von Hohlkörpern (38) mit zwei Öffnungen jener Art, bei der man auf die zu sterilisierenden Flächen ein zuvor verdampftes Sterilisationsmittel aufbringt, **dadurch gekennzeichnet, daß** man die Verdampfung des Mittels außerhalb des Hohlkörpers in der Nähe einer seiner Öffnungen durchführt und mit Hilfe von im Hohlkörper gegenüber der Öffnung mündenden Saugvorrichtungen einen Gasstrom zur Mitführung des verdampften Mittels zu allen zu sterilisierenden Flächen erzeugt, um das Mittel auf die gesamten Flächen zu verteilen.

3. Verfahren nach einem der vorhergehenden Ansprüche, **dadurch gekennzeichnet, daß** das Sterilisationsmittel unter in flüssiger Phase verfügbaren Mitteln, wie zum Beispiel Wasserstoffperoxid, Peressigsäure und dergleichen, ausgewählt wird.

4. Verfahren nach einem der vorhergehenden Ansprüche, **dadurch gekennzeichnet, daß** eine Phase der Entfernung der im Hohlkörper vorhandenen, nicht adhärenten Teilchen oder anderen Elemente der Verdampfungs- und Einführungsphase des Mittels in den Hohlkörper vorausgeht.

5. Verfahren nach Anspruch 4, **dadurch gekennzeichnet, daß** die Entfernungsphase durch Blasen durchgeführt wird.

6. Verfahren nach Anspruch 4, **dadurch gekennzeichnet, daß** die Entfernungsphase durch Saugen durchgeführt wird.

7. Verfahren nach einem der vorhergehenden Ansprüche, **dadurch gekennzeichnet, daß** der Verdampfungsphase nach einer Kontaktzeit des Mittels mit den zu sterilisierenden Flächen eine Phase des Abziehens des verbleibenden Sterilisationsmittels folgt.

8. Verfahren nach Anspruch 7, **dadurch gekennzeichnet, daß** die Abziehphase durch Injizieren eines Abziehmittels in den Innenraum des Hohlkörpers durchgeführt wird.

9. Verfahren nach Anspruch 8, **dadurch gekennzeichnet, daß** das Abziehmittel ein steriles Trocken- oder Heißgas ist.

10. Verfahren nach Anspruch 9, **dadurch gekennzeichnet, daß** das Abziehmittel durch Saugen in den Innenraum des Hohlkörpers mittels im Hohlkörper gegenüber seiner Öffnung wirkenden Saugvorrichtungen mitgeführt wird.

11. Verfahren nach Anspruch 8 oder 9, **dadurch gekennzeichnet, daß** das sterile Gas mittels einer Heißluftdüse injizierte Heißluft ist.

12. Verfahren nach Anspruch 8, **dadurch gekennzeichnet, daß** die Abziehphase mittels einer von einem Brenner injizierten und durch Saugen in den Innenraum des Hohlkörpers mit Hilfe von gegenüber seiner Öffnung darin wirkenden Saugvorrichtungen mitgeführten Flamme durchgeführt wird.

13. Verfahren nach einem der Ansprüche 8 bis 12, **dadurch gekennzeichnet, daß** das Abziehmittel, das Trocken- oder Heißgas bzw. die Flamme auf die Außenseite des Hohlkörpers in der Nähe seiner Öffnung injiziert wird.

14. Einrichtung zur Durchführung des Verfahren nach einem der Ansprüche 1 bis 13, **dadurch gekennzeichnet, daß** sie folgendes umfaßt: Vorrichtungen (1) zur Injektion eines verdampfbaren Sterilisationsmittels, einen Verdampfer (2) gegenüber dem Ausgang der Injektionsvorrichtungen, Mittel zur Erzeugung einer Relativbewegung der Saugvorrichtungen (7, 13; 40, 41) und des Hohlkörpers derart, daß die Saugvorrichtungen im Hohlkörper münden und so darin wirken können, um einen Gasstrom zur Mitführung des verdampften Mittels zu den Innenflächen eines Hohlkörpers (9; 38) zu erzeugen, wenn dieser bezüglich der Einrichtung in Position ist.

15. Einrichtung nach Anspruch 14, **dadurch gekennzeichnet, daß** der Verdampfer in einem Gehäuse (3; 36) eingeschlossen ist, das außerhalb des Hohlkörpers (9; 38) in der Nähe der Öffnung des letzteren angeordnet ist, und mit folgendem versehen ist: einer Öffnung (4) gegenüber dem Ausgang der Injektionsvorrichtungen (1); einem offenen Ende, dessen Form und Innenabmessungen denen der inneren Öffnung des Hohlkörpers (9; 38) im wesentlichen entsprechen.

16. Einrichtung nach Anspruch 14 oder 15, **dadurch gekennzeichnet, daß** die Vorrichtungen (7; 13; 40, 41) zur Erzeugung des Gasstroms aus einem mit einer Saugquelle (13; 41) verbundenen Rohr (7; 40) bestehen.

17. Einrichtung nach Anspruch 16, **dadurch gekennzeichnet, daß** aufgrund der Ausbildung des Hohlkörpers in Form eines Behälters, wobei er somit an einem seiner Enden offen und am anderen geschlossen ist, das Rohr (7) durch die Öffnung des Hohlkörpers eingeführt wird, wobei es ein in der Nähe des Hohlkörperbodens mündendes Ende aufweist und sein zweites, sich auf der Seite des Gehäuses (3) befindendes Ende mit der Saugquelle (13) verbunden ist.

18. Einrichtung nach Anspruch 17, **dadurch gekennzeichnet, daß** das Rohr (7) den Verdampfer (2) und das Gehäuse (3) durchquert.

19. Einrichtung nach Anspruch 16, **dadurch gekennzeichnet, daß** aufgrund der Ausbildung des Hohlkörpers (38) als ein an zwei gegenüberliegenden Enden offenes Rohr oder eine solche Leitung das Saugen durch Anordnung des Saugrohrs (40) an dem Hohlkörperende, das dem Ende, an dem das Injizieren erfolgt, gegenüberliegt, durchgeführt wird.

20. Einrichtung nach einem der Ansprüche 14 bis 19, **dadurch gekennzeichnet, daß** sie Vorrichtungen zum Abziehen des Sterilisationsmittels nach einer Kontaktzeit im Hohlkörper aufweist.

21. Einrichtung nach Anspruch 20, **dadurch gekennzeichnet, daß** die Vorrichtungen einen Generator (16; 21) für steriles Trocken- oder Heißgas enthalten.

22. Einrichtung nach Anspruch 21, **dadurch gekennzeichnet, daß** der Generator ein Brenner (21) ist.

23. Einrichtung nach Anspruch 21 oder 22, **dadurch gekennzeichnet, daß** der Generator (15; 21) außerhalb des Hohlkörpers in der Nähe seiner Öffnung angeordnet ist und Vorrichtungen zum Leiten der Wärme in den Innenraum des Hohlkörpers enthält.

24. Einrichtung nach Anspruch 23, **dadurch gekennzeichnet, daß** die Wärmeleitvorrichtungen aus dem Saugrohr (7; 22; 40) bestehen, dessen Saugende im Hohlkörper in einen von seiner Öffnung entfernten Bereich mündet, um die Wärme in den gesamten Hohlkörper mitzuführen.

25. Einrichtung nach Anspruch 24, **dadurch gekennzeichnet, daß** der Hohlkörper ein Behälter ist und das Rohr (7; 22) durch die Öffnung des letzteren eintritt und in der Nähe seines Bodens mündet.

26. Einrichtung nach Anspruch 24, **dadurch gekennzeichnet, daß** der Hohlkörper ein Rohr oder eine Leitung ist und das Rohr (40) an dem Ende des Hohlkörpers angeordnet ist, das dem gegenüberliegt, in dessen Nähe sich der Wärmegenerator (16; 21) befindet.

27. Einrichtung nach einem der Ansprüche 14 bis 26, **dadurch gekennzeichnet, daß** sie Mittel zur Durchführung einer Entfernung von Staub- oder anderen Teilchen vor der Verdampfungsphase und zur Einbringung des Sterilisationsmittels enthält.

28. Einrichtung nach Anspruch 27, **dadurch gekennzeichnet, daß** die Entfernung durch Saugen und/oder Blasen erfolgt.

29. Einrichtung nach einem der Ansprüche 14 bis 28, **dadurch gekennzeichnet, daß** sie von einer mit Überdruck beaufschlagten Laminarströmung (35) aus sterilem Gas, wie zum Beispiel Luft, durchströmt werden kann.

30. Anlage zur Herstellung und/oder Füllung von Behältern, **dadurch gekennzeichnet, daß** sie eine Einrichtung nach einem der Ansprüche 14 bis 29 aufweist.

## Claims

1. Method for sterilizing hollow bodies (9) having an opening (8), of the kind consisting in depositing a prevaporized sterilizing agent on the surfaces to be sterilized, **characterized in that** it consists in vaporizing the agent outside the hollow body, near its opening, and in causing, with the aid of suction means emerging in the hollow body on the opposite side from the said opening, a gas stream for entraining the vaporized agent towards all of the surfaces to be sterilized, so as to be distribute the agent over the totality of the said surfaces.

2. Method for sterilizing hollow bodies (38) having two openings, of the type consisting in depositing a prevaporized sterilizing agent on the surfaces to be sterilized, **characterized in that** it consists in vaporizing the agent outside the hollow body, near one of its openings and in causing, with the aid of suction means emerging in the hollow body on the opposite side from the said opening, a gas stream for entraining the vaporized agent towards all of the surfaces to be sterilized, so as to distribute the agent over the totality of the said surfaces.

3. Method according to either of the preceding claims, **characterized in that** the sterilizing agent is chosen from agents available in liquid phase, such as hydrogen peroxide, peracetic acid or another agent.

4. Method according to one of the preceding claims, **characterized in that** the phases of vaporizing the agent and of introducing it into the hollow body are preceded by a phase of removing the non-adherent particles or other components present in the hollow body.

5. Method according to Claim 4, **characterized in that** the removal phase is carried out by blowing.

6. Method according to Claim 4, **characterized in that** the removal phase is carried out by suction.

7. Method according to one of the preceding claims, **characterized in that** the vaporization phase is followed, after a time during which the agent is in contact with the surfaces to be sterilized, by a phase of extracting the remaining sterilizing agent.

8. Method according to Claim 7, **characterized in that** the extraction phase is carried out by injecting an extraction agent into the hollow body.

9. Method according to Claim 8, **characterized in that** the extraction agent is a dry or hot sterile gas.

10. Method according to Claim 9, **characterized in that** the extraction agent is entrained, by suction inside the hollow body, with the aid of suction means acting in the hollow body on the opposite side from its opening.

11. Method according to either of Claims 8 and 9, **characterized in that** the sterile gas is hot air injected with the aid of a hot-air nozzle.

12. Method according to Claim 8, **characterized in that** the extraction phase is carried out with the aid of a flame injected by a burner and entrained by suction inside the hollow body, with the aid of suction means acting in the latter, on the opposite side from its opening.

13. Method according to one of Claims 8 to 12, **characterized in that** the extraction agent, dry or hot gas or flame respectively, is injected outside the hollow body, near its opening.

14. Device for implementing the method according to one of Claims 1 to 13, **characterized in that** it comprises means (1) for injecting a vaporizable sterilizing agent, an evaporator (2) facing the outlet of the injection means, means for causing a relative movement between the suction means (7, 13; 40, 41) and the hollow body so that the suction means emerge in the hollow body, so as to act in the latter, in order to cause a gas stream for entraining the vaporized agent towards the internal surfaces of a hollow body (9; 38) when the latter is in place relative to the device.

15. Device according to Claim 14, **characterized in that** the evaporator is contained in a casing (3; 36) located outside the hollow body (9; 38) near the opening of the latter, and is provided with an opening (4) facing the outlet of the injection means (1) and with an open end having a shape and internal dimensions corresponding approximately to those of the internal opening of the hollow body (9; 38).

16. Device according to either of Claims 14 and 15, **characterized in that** the means (7, 13; 40, 41) for causing the gas stream consist of a tube (7; 40) connected to a vacuum source (13; 41).

17. Device according to Claim 16, **characterized in that**, the hollow body being in the form of a container, that is to say open at one of its ends and closed at the other, the tube (7) is introduced via the opening of the hollow body, the tube has an end emerging near the bottom of the hollow body and its second end, located on the same side as the casing (3), is connected to the vacuum source (13).

18. Device according to Claim 17, **characterized in that** the tube (7) passes through the evaporator (2) and the casing (3).

19. Device according to Claim 16, **characterized in that**, the hollow body (38) being a tube or a duct open at the two opposed ends, the suction is achieved by placing the suction tube (40) at that end of the hollow body which is on the opposite side from that where the injection takes place.

20. Device according to one of Claims 14 to 19, **characterized in that** it includes means for removing the sterilizing agent after a contact time in the hollow body.

21. Device according to Claim 20, **characterized in that** the means comprise a generator (16; 21) for generating a dry or hot sterile gas.

22. Device according to Claim 21, **characterized in that** the generator is a burner (21).

23. Device according to either of Claims 21 and 22, **characterized in that** the generator (16; 21) is located outside the hollow body, near its opening, and it includes means for directing the heat into the hollow body.

24. Device according to Claim 23, **characterized in that** the means for directing the heat consist of the suction tube (7; 22; 40), the suction end of which emerges in the hollow body in a region remote from its opening, so as to entrain the heat into the entire hollow body.

25. Device according to Claim 24, **characterized in that**, the hollow body being a container, the tube (7; 22) penetrates via the opening of the latter and emerges near its bottom.

26. Device according to Claim 24, **characterized in that**, the hollow body being a tube or a duct, the tube (40) is located at that end of the hollow body on the opposite side from that close to which the heat generator (16; 21) is located.

27. Device according to one of Claims 14 to 26, **characterized in that** it includes means for removing the dust or other particles prior to the phase of vaporizing and injecting the sterilizing agent.

28. Device according to Claim 27, **characterized in that** the removal is carried out by suction and/or blowing.

29. Device according to one of Claims 14 to 28, **characterized in that** a laminar flow (35) of overpressurized sterile gas, such as air, can pass through it.

30. Plant for manufacturing and/or for filling containers, **characterized in that** it includes a device according to one of Claims 14 to 29.
